# EUROPEAN PATENT APPLICATION

(11) **EP 4 224 416 A1**
(43) Date of publication of application: **09.08.2023**
(21) Application number: 22155682.2
(22) Date of filing: 08.02.2022
(51) Int. Cl.: G06T 7/00, G06T 7/136, A61B 6/00

(54) **IDENTIFYING ANGIOGRAPHIC IMAGES FOR VESSEL ASSESSMENT**

(71) Applicant: Koninklijke Philips N.V., 5656 AG Eindhoven (NL)
(72) Inventor: VAN LAVIEREN, Martijn Anne, Eindhoven (NL); SPOEL, Cornelis Willem Johannes Immanuël, Eindhoven (NL)
(74) Representative: Philips Intellectual Property & Standards

(57) **Abstract**

A system for identifying an angiographic image for assessing a vessel is provided. The system includes one or more processors configured to: receive angiographic image data comprising one or more temporal sequences of images representing a flow of an injected contrast agent through the vessel; segment the vessel in the images; analyse the images to determine a contrast agent intensity variation along a portion of a length of the segmented vessel; and calculate a vessel overlap metric for the vessel in the image. The vessel overlap metric quantifies a magnitude of overlap between the vessel and one or more further vessels represented in the image. One of the plurality of images is identified for assessing the vessel, based on a comparison of the calculated vessel overlap metrics.

## Description

### Technical Field

The present disclosure relates to identifying an angiographic image for assessing a vessel. A system, a computer-implemented method, and a computer program product, are disclosed.

### Background

Angiographic images are routinely generated in the context of clinical investigations. Such images are often generated as temporal sequences that represent a flow of an injected contrast agent. The images are often used to perform an assessment of a vessel. For example, they may be used to calculate a value of a blood flow parameter for the vessel, or to make geometric measurements on a stenosis in a vessel.

However, it can be challenging to manually select images with which to perform such assessments. Clinical investigations often involve the generation of a large number of images in a temporal sequence. Selecting an appropriate image from a set of temporal sequences can therefore be time-consuming. Moreover, the vasculature can move, and also deform, during the time in which the temporal sequence is acquired. This makes it difficult to keep track of a vessel of interest in order to select an appropriate image of the vessel.

By way of an example, a temporal sequence of images of a coronary artery may be acquired during a coronary angiography in order to perform a clinical assessment for coronary artery disease "CAD". The coronary arteries typically undergo significant motion during the time in which the temporal sequence is acquired. They also deform in accordance with the beating heart. Selecting an image from a temporal sequence that clearly depicts a coronary artery is important in performing assessments of the vessel. When images are used for such assessments, motion, and also vessel deformation, may result in a misleading view that confounds its assessment.

Consequently, there is a need for improvements in identifying angiographic images for use in assessing vessels.

### Summary

According to one aspect of the present disclosure, a system for identifying an angiographic image for assessing a vessel, is provided. The system includes one or more processors configured to:
- receive angiographic image data comprising one or more temporal sequences of images representing a flow of an injected contrast agent through the vessel; and
- for a plurality of images in the angiographic image data:
   segment the vessel in the image;
   analyse the image to determine a contrast agent intensity variation along a portion of a length of the segmented vessel; and
   calculate, based on the contrast agent intensity variation, a vessel overlap metric for the vessel in the image, the vessel overlap metric quantifying a magnitude of overlap between the vessel and one or more further vessels represented in the image; and
   wherein the one or more processors are further configured to:
- identify one of the plurality of images for assessing the vessel, based on a comparison of the calculated vessel overlap metrics.

The vessel overlap metric has been found to be a reliable indicator for selecting an image for assessing a vessel because vessels having a large amount of overlap with other vessels in the image exhibit intensity variations along their length that can adversely impact assessments such as the measurement of vessel geometry and blood flow parameters. Vessels having a large amount of overlap with other vessels can falsely suggest the presence of branches in the vessel, likewise confounding such assessments. By identifying an image for assessing the vessel based on its vessel overlap metric, an image with a low vessel overlap metric may be used in such assessments, i.e. an image in which the vessel has a small amount of overlap, or even no overlap with other vessels. This in turn facilitates a more reliable assessment of the vessel. Such assessment may include at least one of identifying a stenosis in the vessel, calculating a value of a blood flow parameters such as the fractional flow reserve "FFR", the instantaneous wave-free ratio "iFR" and/or microvascular resistance "MVR" .

Further aspects, features, and advantages of the present disclosure will become apparent from the following description of examples, which is made with reference to the accompanying drawings.

### Brief Description of the Drawings

Fig. 1 is a schematic diagram illustrating an example of a system 100 for identifying an angiographic image for assessing a vessel, in accordance with some aspects of the present disclosure.
Fig. 2 is a flowchart illustrating an example of a method of identifying an angiographic image for assessing a vessel, in accordance with some aspects of the present disclosure.
Fig. 3 is a schematic diagram illustrating a heart, including an example of a vessel 120, in accordance with some aspects of the present disclosure.
Fig. 4 is an example of an angiographic image 110 including a segmented vessel 140, in accordance with some aspects of the present disclosure.
Fig. 5 is an example of an angiographic image including a vessel 120 and an overlap region 150 in which the vessel overlaps with another vessel, in accordance with some aspects of the present disclosure.
Fig. 6 is an example of an angiographic image including a segmented vessel 140, and a centerline 160 of the vessel, in accordance with some aspects of the present disclosure.
Fig. 7 is a schematic diagram illustrating a) a vessel 120 including an overlap region 150 in which the vessel overlaps with another vessel, and b) a graph representing a contrast agent intensity INT variation along a portion of the length of the vessel, in accordance with some aspects of the present disclosure.

### Detailed Description

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer implemented method, and in a computer program product, in a corresponding manner.

In the following description, reference is made to a system for identifying an angiographic image for assessing a vessel. Reference is made to examples in which the vessel is a coronary artery. However, it is also to be appreciated that the vessel may alternatively be a coronary vein. In other words, the vessel may be a coronary vessel. More generally, the system may be used to identify angiographic images for assessing a vessel, i.e. a vein or an artery, from other regions of the body than the heart, such as organs like liver, brain, kidney, limbs.

Reference is also made herein to examples in which angiographic images are identified for assessing a vessel for the purpose of calculating a value of a blood flow parameter, or a value of a geometric measurement, for the vessel. However, it is to be appreciated that these serve only as examples, and that the system and method disclosed herein may alternatively be used to identify angiographic images for assessing vessels for other purposes.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD

As mentioned above, it can be challenging to manually select images for use in assessing a vessel.

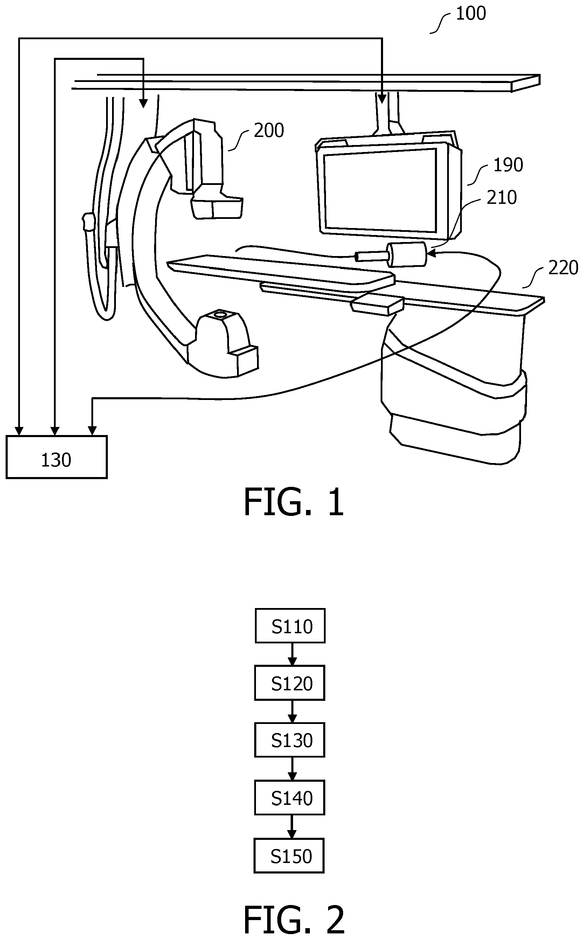

Fig. 1 is a schematic diagram illustrating an example of a system 100 for identifying an angiographic image for assessing a vessel, in accordance with some aspects of the present disclosure. Fig. 2 is a flowchart illustrating an example of a method of identifying an angiographic image for assessing a vessel, in accordance with some aspects of the present disclosure. Operations described in relation to the system 100 illustrated in Fig. 1, may also be performed in the method illustrated in Fig. 2, and vice versa. With reference to Fig. 1 and Fig. 2, the example system 100 includes one or more processors 130 configured to:
- receive S110 angiographic image data comprising one or more temporal sequences of images representing a flow of an injected contrast agent through the vessel 120; and
- for a plurality of images in the angiographic image data:
   segment S120 the vessel 120 in the image;
   analyse S130 the image to determine a contrast agent intensity INT variation along a portion of a length of the segmented vessel 140; and
   calculate S140, based on the contrast agent intensity INT variation, a vessel overlap metric for the vessel in the image, the vessel overlap metric quantifying a magnitude of overlap between the vessel 120 and one or more further vessels represented in the image; and
- wherein the one or more processors 130 are further configured to
- identify S150 one of the plurality of images 110 for assessing the vessel 120, based on a comparison of the calculated vessel overlap metrics.

The vessel overlap metric has been found to be a reliable indicator for selecting an image for assessing a vessel because vessels having a large amount of overlap with other vessels in the image exhibit intensity variations along their length that can adversely impact assessments such as the measurement of vessel geometry and blood flow parameters. Vessels having a large amount of overlap with other vessels can falsely suggest the presence of branches in the vessel, likewise confounding such assessments. By identifying an image for assessing the vessel based on its vessel overlap metric, an image with a low vessel overlap metric may be used in such assessments, i.e. an image in which the vessel has a small amount of overlap, or even no overlap with other vessels. This in turn facilitates a more reliable assessment of the vessel. Such assessment may include at least one of identifying a stenosis in the vessel, calculating a value of a blood flow parameters such as the fractional flow reserve "FFR", the instantaneous wave-free ratio "iFR", Coronary Flow Reserve "CFR" and/or microvascular resistance "MVR", such as disclosed in US patent no. 10342442, WO 2020/053099 A1, European patent applications no. 21290062.5 and 21290061.7, herein incorporated by reference.

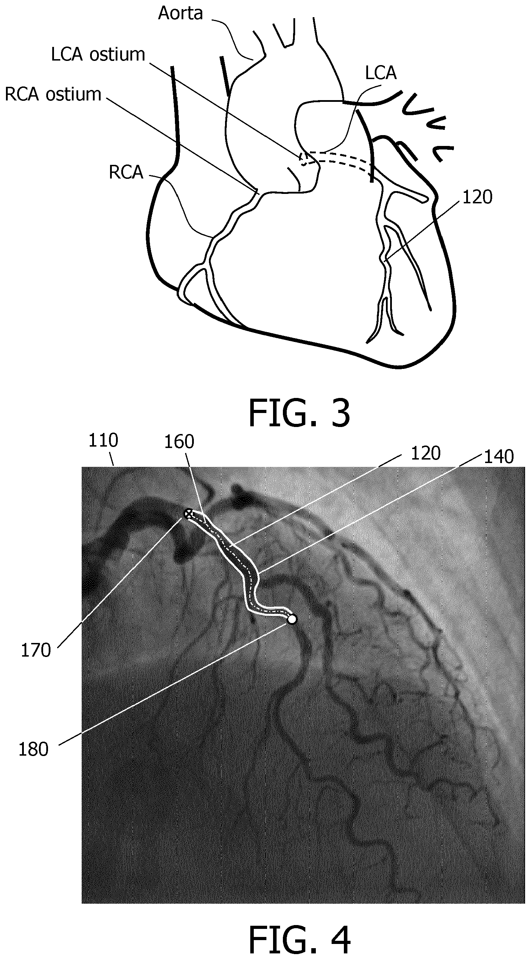

Fig. 3 is a schematic diagram illustrating a heart, including an example of a vessel 120, in accordance with some aspects of the present disclosure. The heart illustrated in Fig. 3 is labelled with the left coronary artery, LCA, the right coronary artery, RCA, and the LCA ostium and RCA ostium that respectively define the openings of these arteries in the aorta. By way of an example, the operations described above with reference to Fig. 1 and Fig. 2 may be performed in order to identify an angiographic image for assessing the left coronary artery, LCA, i.e. the vessel 120 illustrated in Fig. 3.

With reference to Fig. 2, in the operation S110, angiographic image data comprising one or more temporal sequences of images, is received. The angiographic image data may be received by the one or more processors 130 illustrated in Fig. 1. The angiographic data may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals. In general, the one or more processors 130 may receive the angiographic image data from an angiographic imaging system, or from another source such as a computer readable storage medium, the Internet, or the Cloud, for example.

The angiographic image data that is received in the operation S110 includes one or more temporal sequences of images representing a flow of an injected contrast agent through the vessel 120. In general, the temporal sequence(s) of images may be generated by an angiographic imaging system such as a projection X-ray imaging system or a volumetric imaging system. Projection X-ray imaging systems typically include a support arm such as a so-called "C-arm", or an "O-arm", that supports an X-ray source-detector arrangement. Projection X-ray imaging systems may alternatively include a support arm with a different shape to these examples. Projection X-ray imaging systems typically generate projection X-ray images with the support arm held in a static position with respect to an imaging region during the acquisition of image data. In some examples, the temporal sequence of images may be fluoroscopic, i.e. live images. In some examples, temporal sequence of images may be generated using a digital subtraction angiography "DSA" technique, and wherein each image is generated by subtracting from the image the corresponding pixel intensities of a background image. The temporal sequence(s) of images may be generated by the projection X-ray imaging system 200 illustrated in Fig. 1, for example. By way of an example, the temporal sequence of images may be generated by the Philips Azurion 7 X-ray imaging system, which is an example of a projection X-ray imaging system that is marketed by Philips Healthcare, Best, The Netherlands.

As mentioned above, the temporal sequence(s) of images that are received in the operation S110 may alternatively be generated by a volumetric imaging system. The volumetric imaging system may be a volumetric X-ray imaging system, or an MRI imaging system, for example. A volumetric X-ray imaging system typically generates image data whilst rotating, or stepping, an X-ray source-detector arrangement around an imaging region, and subsequently reconstructs the image data obtained from multiple rotational angles into a 3D, or volumetric image. Examples of volumetric X-ray imaging systems include computed tomography "CT" imaging systems, cone beam CT "CBCT" imaging systems, and spectral CT imaging systems. In some examples, the temporal sequence of images may be fluoroscopic, i.e. live images. In some examples, the temporal sequence of images may be generated from a volumetric X-ray imaging system using a digital subtraction angiography "DSA" technique.

Alternatively, the temporal sequence(s) of images that are received in the operation S110 may be generated by a magnetic resonance imaging "MRI" system. For example, magnetic resonance angiography "MRA" volumetric image data may be generated by injecting a contrast agent into the vasculature and using oscillating magnetic fields at specific resonance frequencies to generate images of various anatomical structures using an MRI imaging system.

The temporal sequence(s) of images that are received in the operation S110 may in general be generated subsequent to the injection of a contrast agent into a vasculature that comprises the vessel. The contrast agent may include a substance such as Iodine, or a Lanthanide such as Gadolinium, or indeed another substance that provides visibility of a flow in the vasculature into which the contrast agent is injected. The contrast agent may be injected into the vasculature from a syringe. The syringe may be controlled manually, or by a syringe driver. Together, the syringe and the syringe driver may be referred-to as an injector. An injection catheter is typically used to fluidically couple the syringe to the vasculature. In-use, a distal end of the injection catheter is inserted into the vasculature in order to inject the contrast agent. By way of an example, the contrast agent may be injected into the vasculature using the injector 210 illustrated in Fig. 1. The injector 210 may be controlled manually, or automatically.

As may be appreciated, a vessel represented in a temporal sequence of angiographic images may undergo motion, and also deformation over time. Typical sources of motion and deformation in a subject include cardiac motion, respiratory motion, and gross movement of the subject. With reference to the heart illustrated in Fig. 3, temporal sequences of images of the coronary arteries typically undergo significant changes over time as a result of cardiac motion. As a consequence, both their position and shape change over time in a temporal sequence of angiographic images. This makes it difficult to track their position over time in a temporal sequence. A further consequence of this motion is that the degree of overlap between a vessel, such as for example the left coronary artery, and other vessels in these images, changes over time. Such overlaps can confound an assessment of the vessel. Similar challenges occur when performing assessments on angiographic images of other vessels in the vasculature. The inventors have found that the method described with reference to Fig. 1 and Fig. 2 can be used to reliably identify an image from a temporal sequence of images for use in assessing a vessel.

With continued reference to Fig. 2, in the operation S120, a vessel 120 is segmented. In this operation, the vessel may be segmented in one or more images in a single sequence, or in one or more images in multiple sequences. Various image segmentation techniques are known for this purpose, including thresholding, template matching, active contour modelling, model-based segmentation, neural networks, e.g., U-Nets, and so forth. Fig. 4 is an example of an angiographic image 110 including a segmented vessel 140, in accordance with some aspects of the present disclosure. The image in Fig. 4 represents a portion of the coronary vasculature, and includes a vessel 120, which in this example is the left coronary artery. In Fig. 4, the vessel 120 is segmented by delineating its border along a portion of its length, thereby providing segmented vessel 140.

With continued reference to Fig. 2, in the operation S130, the image with the segmented vessel is analysed to determine a contrast agent intensity INT variation along a portion of a length of the segmented vessel 140. In this regard, the contrast agent intensity INT variation may be determined along the entire portion of the length of the segmented vessel 140, or along less than the entire portion of the length of the segmented vessel 140. Various techniques may be used in this operation. In one technique, the segmented vessel may be straightened-out using image processing techniques, and an average of the image intensity values across the vessel may be calculated at positions along the vessel to provide the contrast agent intensity INT variation. Alternative techniques may include determining a centerline of the vessel in the images. In one example, the one or more processors 130 analyse S 130 the images to determine a contrast agent intensity INT variation along a portion of a length of the segmented vessel 140, by:
- determining a centerline 160 of the vessel in the image; and
- determining image intensity values at a plurality of positions along the centerline 160.

In this example, the position of the centerline may be determined as the mid-point between the bilateral borders of the segmented vessel. The position of the centerline may alternatively be determined in the segmented vessel using a Fast Marching algorithm. A dynamic programming technique may be used to optimise the position of the centerline with respect to the border of the segmented vessel. Fig. 4 includes an example of a centerline 160 of the segmented vessel 140 that may be determined in this manner. In this example, the image intensity values may be determined at positions along the centerline 160 by calculating an image intensity value at the positions along the centerline based on:
- the image intensity value at the position; or
- an average of the image intensity values within the segmented vessel 140 along a line transversely intersecting the centerline at the position; or
- a maximum of the image intensity values within the segmented vessel 140 along a line transversely intersecting the centerline at the position.

The positions along the centerline 160 may be defined ad-hoc, or at regular intervals. For example, the positions may be defined every n pixels along the vessel, or at another spacing, such as every millimetre along the vessel.

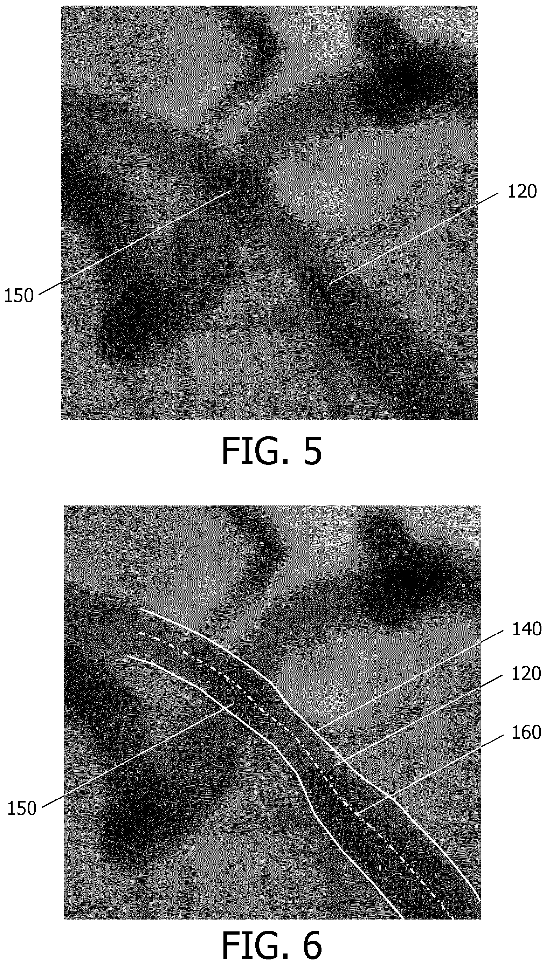

An example of a contrast agent intensity INT variation that is determined in this manner is now described with reference to Fig. 5 - Fig. 7. Fig. 5 is an example of an angiographic image including a vessel 120 and an overlap region 150 in which the vessel overlaps with another vessel, in accordance with some aspects of the present disclosure. Fig. 5 illustrates in more detail a portion of the angiographic image illustrated in Fig. 4. As can be seen in Fig. 5, within the overlap region 150, vessel 120 overlaps with another vessel, and the black-level intensity of the contrast agent is increased. As may be appreciated, the overlap region 150 can confound analysis of the vessel. For example, the change in black level intensity in this region can affect measurements of the vessel's geometry. It can also affect angiographically-determined measurements of blood flow in the vessel. Fig. 6 is an example of an angiographic image including a segmented vessel 140, and a centerline 160 of the vessel, in accordance with some aspects of the present disclosure. In Fig. 6, the vessel 120 from Fig. 5 has been segmented by delineating its bilateral borders along a portion of its length, thereby providing segmented vessel 140.

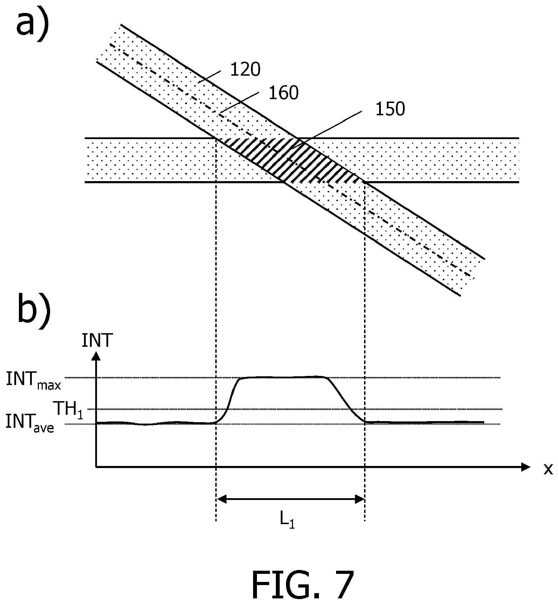

Fig. 7 is a schematic diagram illustrating a) a vessel 120 including an overlap region 150 in which the vessel overlaps with another vessel, and b) a graph representing a contrast agent intensity INT variation along a portion of the length of the vessel, in accordance with some aspects of the present disclosure. Fig. 7a) is a schematic depiction of the vessel 120 illustrated in Fig. 5 and Fig. 6. In Fig. 7b), the contrast agent intensity INT in the graph has a higher value in the overlap region 150 than outside this region.

Returning to Fig. 2, in the operation S140, a vessel overlap metric is calculated for the vessel in the image based on the contrast agent intensity INT variation. The vessel overlap metric quantifies a magnitude of overlap between the vessel 120 and one or more further vessels represented in the image.

In one example, the vessel overlap metric is calculated by:
- comparing the contrast agent intensity INT variation along the portion of the length of the segmented vessel 140 with one or more reference contrast agent intensity values.

In one example, the vessel contrast agent intensity INT variation is normalised to reference contrast agent intensity value and used to provide a vessel overlap metric in the form of a probability of portions of the vessel overlapping other vessels. In this example, the reference contrast agent intensity value may be a mean value. This example is described with reference to Fig. 7, an wherein the vessel contrast agent intensity INT has an average value INTₐᵥₑ outside the overlap region 150, and a maximum value INTₘₐₓ within the overlap region 150. In this example, the contrast agent intensity INT may be normalised to the average value INTₐᵥₑ outside the overlap region 150, and used to provide a vessel overlap metric in the form of a probability of portions of the vessel overlapping other vessels.

In another example, a vessel overlap metric is calculated as a digital value by using a reference contrast agent intensity value as a threshold value. This example is also described with reference to Fig. 7. In this example, the vessel contrast agent intensity INT is compared with a reference contrast agent intensity value TH₁. In this example, sections of the vessel having contrast agent intensity INT values that exceed the reference contrast agent intensity value TH₁ are assigned as overlap regions, whereas sections of the vessel within which the contrast agent intensity INT is less than the reference contrast agent intensity value TH₁ are assigned as non-overlap regions. The value of the reference contrast agent intensity value TH₁ may be determined in various ways. For example, it may be set based on the average of the contrast agent intensity INT values along the portion of the segmented vessel. For instance, the reference contrast agent intensity value TH₁ may be set to a value that is e.g. 20 % higher than the average of the contrast agent intensity INT values along the portion of the segmented vessel.

In a related example, the one or more processors 130 are configured to:
- identify one or more overlap regions 150 along the portion of the length of the segmented vessel 140 having contrast agent intensity values that exceed a reference contrast agent intensity value TH₁; and
- calculate the vessel overlap metric based on the identified one or more overlap regions 150.

In this example, the vessel overlap metric for the vessel in the image may be calculated based on:
- a total length L₁ of the one or more overlap regions for the vessel 120; or
- a maximum contrast agent intensity value INTₘₐₓ within the one or more overlap regions for the vessel 120; or
- a count of the number of overlap regions for the vessel 120.

By way of an example, the total length L₁ of a single overlap region for a vessel is illustrated in Fig. 7. The total length L₁ of a single overlap region may be short for a vessel that overlaps with another vessel having a relatively smaller diameter. Similarly, the total length L₁ of a single overlap region may be short for a vessel that overlaps with another vessel at a near-perpendicular angle. These situations may result in an overlap metric with a relatively low value. By contrast, the total length L₁ of an overlap region may be longer for a vessel that overlaps with another vessel having a relatively larger diameter. Similarly, the total length L₁ of an overlap region may be longer for a vessel that overlaps with another vessel at a near-parallel angle. These situations may result in an overlap metric with a relatively higher value in view of the increased potential for these situations to confound assessment of the vessel. If a vessel overlaps with multiple vessels, the overlap metric may be determined from the combined length of the individual overlap regions.

By way of another example, the vessel overlap metric may alternatively be calculated based on a maximum contrast agent intensity value INTₘₐₓ within the one or more overlap regions for the vessel. The maximum contrast agent intensity value INTₘₐₓ for a vessel may be indicative of the total number of overlapping vessels at a common location. If more than two vessels overlap at a common location, there may be an elevated risk of errors when assessing the vessel, and thus using the maximum contrast agent intensity value INTₘₐₓ to determine the vessel overlap metric reflects this increased potential for errors.

By way of another example, the vessel overlap metric may alternatively be calculated based on a count of the number of overlap regions for the vessel 120. The presence of multiple overlap regions in a vessel also increases the risk of errors when assessing the vessel. The presence of multiple overlap regions elevates the risk of confounding a determination of the path of a vessel, as well as geometric measurements of a vessel such as its diameter. Thus using a count of the number of overlap regions to determine the vessel overlap metric reflects this elevated potential for errors.

In any of these three examples, confidence values may also be assigned to the contrast agent intensity values; and the vessel overlap metric for the vessel in the image may be calculated based further on the confidence values. Confidence values may be calculated based on the amount of noise in the contrast agent intensity INT. For example, high confidence values may be assigned to portions of the vessel in which the contrast agent intensity INT variation has relatively low amounts of noise, whereas low confidence values may be assigned to portions of the vessel in which the contrast agent intensity INT variation has relatively higher amounts of noise. Confidence values may also be generated by a neural network. For example, if the segmentation described above in the operation S120 is performed by a neural network, the neural network may generate confidence values that represent the accuracy of the segmented vessel 140. The confidence values may be used to calculate the vessel overlap metric by assigning as overlap regions, only the portions of the vessel for which the confidence values indicate that the vessel is actually an overlap region with a high degree of certainty.

Returning to Fig. 2, in the operation S150, one of the plurality of images 110 is identified for assessing the vessel 120, based on a comparison of the calculated vessel overlap metrics. Typically, the image 110 having the lowest vessel overlap metric is identified for use in assessing the vessel. This may corresponds to a vessel that has the lowest amount of overlap with other vessels, or even no overlap at all. Various techniques are contemplated for identifying the image, including displaying the image, highlighting the image, outputting a reference to the image, and so forth. The identified image may be displayed on a monitor, such as the monitor 190 illustrated in Fig. 1, for example.

In one example, the image is displayed, and the one or more processors 130 are further configured to:
- indicate in the displayed image 110 a location of one or more overlap regions 150 of the vessel at which the vessel overlaps with one or more further vessels represented in the image.

The location of the overlap region(s) is determined in the operation S140 described above. By way of some examples, the location may be indicated by highlighting the relevant location in the image, adding a marker, and so forth. Identifying the location of the overlap region may assist an operator in assessing the suitability of the image for the assessment. For example, given such information on a location of the overlap region(s), the operator may decide to perform an assessment on a portion of the vessel that avoids the overlap region(s).

The result of the operations described above with reference to Fig. 2 is thus to identify S150 an image 110 for assessing the vessel 120. In this regard, various assessments, or more specifically "angiographic assessments" may be performed on the vessel.

In some examples, the one or more processors 130 may calculate a value of a blood flow parameter for the vessel 120 using the image. An example of a blood flow parameter that may be calculated from an image that is identified in this manner is a transit time. The transit time is defined as the time taken for an injected bolus of contrast agent to travel between a proximal position in the vessel, and a distal position in the vessel. An angiographic determination of the transit time involves the selection of angiographic images in which a front of the injected contrast agent bolus is respectively in a proximal position in a vessel, i.e. proximal to the ostium of the relevant vessel, and in a distal position in the vessel, i.e. distal to the relevant ostium. The selection of each of these images may be facilitated using the method described with reference to Fig. 2, in particular by providing images with a reduced risk that an overlap between the vessel and other vessels in the image confounds a determination of the position of the front of the contrast agent or the analysis of a stenosis in the image.

Other examples of blood flow parameters that may be calculated from an image that is identified in the manner described with reference to Fig. 2 include the Fractional Flow Reserve "FFR", the Coronary Flow Reserve "CFR", the Thrombolysis in Myocardial Infarction "TIMI" flow grade, and the Index of Microcirculatory Resistance "IMR" or Microvascular Resistance "MVR". Values for these parameters may be determined by using the identified angiographic image as an input to a haemodynamic model. These blood flow parameters have prognostic value as indicators of Coronary Artery Diseases "CAD". By way of an example, a technique for calculating a Fractional Flow Reserve value for a blood vessel from angiographic images is disclosed in PCT patent application WO 2016/087396 A1.

Another example of a blood flow parameter that may be calculated from an angiographic image that is identified in this manner is a temporal velocity profile of a contrast agent injected into a vessel. The temporal nature of the velocity profile provides additional information on the vessel as compared to a measurement of only a single velocity, such as the average or maximum velocity, as may be derived from the transit time. A technique for making an angiographic measurement of the temporal velocity profile is described in a document by Pereira V. M., et al., "A DSA-based Method Using Contrast-Motion Estimation for the Assessment of the Intra-Aneurysmal Flow Changes Induced by Flow-Diverter Stents", American Journal of Neuroradiology, 2013, 34(4), pages 808-815. This technique relies upon a determination of a 2D plot of the contrast agent intensity along a length of the vessel over time. This plot may be generated by analysing the contrast agent intensity in successive images from a temporal sequence of angiographic images of the vessel. By selecting images for use in such analysis in accordance with the method described with reference to Fig. 2, images, or sequences of images, having high values of vessel overlap metric may be omitted, and thus the reliability of the temporal velocity profile may be improved.

Other types of assessment may also be performed on the vessel in images that are identified in accordance with the method described with reference to Fig. 2. For example, the one or more processors 130 may alternatively or additionally calculate a value of a geometric measurement of the vessel 120 in the image. Examples of geometric measurements of the vessel that may be calculated include a length, or a diameter of a vessel or a stenosis in the image, and a minimum lumen diameter. Known image processing techniques may be used to identify such features, and also to measure their values. For example, a feature detector may be designed, or a neural network may be trained, and used to identify a vessel of interest in the angiographic images. By identifying an image in the above manner in order to make such geometric measurements, the reliability of the measurements may be improved because the method identifies an image based on the vessel overlap metric, and thus provides an image with fewer confounding features.

One or more further operations may also be performed by the system described with reference to Fig. 1, and likewise in the method described with reference to Fig. 2.

In one example, the position of a centerline is determined in an image in a temporal sequence, and then mapped to other images in the temporal sequence. In this example, the one or more processors 130 are configured to segment the vessel in the images in the angiographic image data by:
- determining a centerline 160 of the vessel in at least one of the images in the one or more temporal sequences of images; and
- mapping the centerline 160 to one or more further images in the one or more temporal sequences of images.

In this example, the position of the centerline may be determined in an image as described above. Mapping the centerline to the one or more further images in the one or more temporal sequences of images in this manner, alleviates the challenge of segmenting the image into which it is mapped, particularly when the vessel undergoes motion and deformation in the time interval between the images. For example, if the centerlines is mapped between successive images, the mapped centerline from one image may serve as an initial position to start the segmentation in the next image. This may help to speed-up the segmentation operation.

In a related example, the operation of determining a centerline 160 includes:
- determining a centerline of the vessel in a plurality of images in the one or more temporal sequences of images, and wherein each centerline represents a different point in time in the cardiac cycle; and
- wherein the mapping the centerline 160 comprises mapping each centerline to the one or more further images in the one or more temporal sequences of images representing the same point in time in the cardiac cycle.

Mapping the centerlines in this manner alleviates the challenge of segmenting the vessel because the mapped centerlines may serve as an initial position to start the segmentation in the images into which they are mapped. Since both images represent the same point in time in the cardiac cycle, the vessel is expected to be in a similar position in both images. In this example, the point in time in the cardiac cycle may be any point in time in the cardiac cycle. By way of an example, it may be a biologically-defined phase such as the start of the systolic phase, or the start of the diastolic phase. The point in time in the cardiac cycle may be determined for the images in the temporal sequence(s) using various sensors, including electrocardiogram "ECG" sensors, and blood pressure sensors. The signals generated by such sensors may include a signature that is indicative of a biologically-defined phase. The point in time in the cardiac cycle may alternatively be determined from the angiographic images in the temporal sequence(s) themselves. In this respect, it is contemplated to analyse the angiographic images in the one or more temporal sequences, and to detect the pulsation of an image feature such as a coronary artery, or the distal end of a catheter of a contrast agent injector, and to derive a signal representing the cardiac cycle from these features. For example, a dimension of the coronary arteries may be detected in the angiographic images and used to provide a signal indicative of the cardiac cycle. A shrinking of the coronary arteries in such images corresponds to the systolic phase. A document by Ciusdel C., et al., "Deep neural networks for ECG-free cardiac phase and end-diastolic frame detection on coronary angiographies", Comput Med Imaging Graph. 2020 Sep;84:101749, discloses techniques that may be used for this purpose.

In another related example, a start position and an end position are used to define the portion of a vessel of interest in the images. In this example, the one or more processors 130 are configured to:
- display an image in which the centerline 160 is determined;
- receive input defining a start position 170, and an end position 170, of the portion of the vessel in the displayed image; and
- map the start position 170 and the end position 180 from the displayed image to the one or more further images in the one or more temporal sequences of images to define the portion of the length of the segmented vessel 140 in said images.

This example alleviates the challenge of tracking the portion of the vessel of interest between images in the temporal sequence(s) by automatically mapping the start and end positions into the images in the temporal sequence(s). In this example, the input defining the start position 170 and the end position 180 in the displayed image may be received from a user input device configured to receive user input, such as a keyboard, a mouse, a touchscreen, and so forth. Alternatively, the start position and the end position may be defined automatically in the displayed image. For example, a feature detector may be designed, or a neural network may be trained, and used to identify a vessel of interest in the angiographic images, and to define the start and end positions based on angiographic training images labelled with preferred locations for these positions. The start and end positions may also be defined with respect to a stenosis in the images. In this regard, the feature detector, or the neural network may for example be trained to detect a narrowing in a vessel that is indicative of a stenosis. Subsequently, the start and end positions may be defined with respect to the stenosis.

In another example, images corresponding to a common point in time in the cardiac cycle are identified, and the method described with reference to Fig. 1 and Fig. 2 is performed selectively with these images. In this example, the one or more processors 130 are configured to identify, from the angiographic image data, a plurality of images corresponding to a common point in time in the cardiac cycle, and the operations of:
- segmenting S120 the vessel;
- analysing S130 the image to determine a contrast agent intensity INT variation along a portion of a length of the segmented vessel 140; and
- calculating S 140, based on the contrast agent intensity INT variation, a vessel overlap metric for the vessel in the image, the vessel overlap metric quantifying a magnitude of overlap between the vessel 120 and one or more further vessels represented in the image;
are performed for the identified plurality of images.

In this example, the point in time in the cardiac cycle may be determined by using a sensor, or alternatively by analysing the images. Suitable techniques for identifying the point in time in the cardiac cycle using a sensor, or from the angiographic images themselves, are described above. Using the point in time in the cardiac cycle to select images in this manner simplifies the operations of segmenting S120, analysing S130, and calculating S140 that are described above with reference to Fig. 2. Moreover, it may be desired to perform an assessment on the vessel at a particular point in time in the cardiac cycle. This example may be used to provide, and selectively process the desired images whilst avoiding the processing of superfluous images.

In another example, a subset of the angiographic images from the temporal sequence(s) images having contrast agent along at least the portion of the length of the segmented vessel, are identified, and the method described with reference to Fig. 1 and Fig. 2 is performed with these images. In this example, the one or more processors 130 are configured to identify, from the angiographic image data, a plurality of images having contrast agent along at least the portion of the length of the segmented vessel 140, and the operations of:
segmenting S120 the vessel;
analysing S130 the image to determine a contrast agent intensity INT variation along a portion of a length of the segmented vessel 140; and
calculating S140, based on the contrast agent intensity INT variation, a vessel overlap metric for the vessel in the image, the vessel overlap metric quantifying a magnitude of overlap between the vessel 120 and one or more further vessels represented in the image;
are performed for the identified plurality of images.

In this example, the presence of contrast agent along at least the portion of the length of the segmented vessel may be determined by detecting a position of a front of the contrast agent in the images. This may be performed by analysing the segmented images and detecting the position of the front based on its movement between successive images. Using the presence of contrast agent to select images in this manner simplifies the processing of the images by avoiding that the operations of segmenting S120, analysing S130, and calculating S140, are performed on images that are not required for assessing the vessel.

It is noted that in the examples described above, the system 100 may also include one or more of: an angiographic imaging system for providing the angiographic image data, such as for example the projection X-ray imaging system 200 illustrated in Fig. 1; an injector 210 for injecting a contrast agent; a monitor 190 for displaying the identified angiographic image, temporal sequence(s) of angiographic images, the results of the assessment of the vessel, and so forth; a patient bed 220; and a user input device configured to receive user input (not illustrated in Fig. 1), such as a keyboard, a mouse, a touchscreen, and so forth.

In another example, a computer-implemented method of identifying an angiographic image 110 for assessing a vessel 120, is provided. The method comprises:
- receiving S110 angiographic image data comprising one or more temporal sequences of images representing a flow of an injected contrast agent through the vessel 120; and
- for a plurality of images in the angiographic image data:
   segmenting S120 the vessel 120 in the image;
   analysing S130 the image to determine a contrast agent intensity INT variation along a portion of a length of the segmented vessel 140; and
   calculating S140, based on the contrast agent intensity INT variation, a vessel overlap metric for the vessel in the image, the vessel overlap metric quantifying a magnitude of overlap between the vessel 120 and one or more further vessels represented in the image.

The method further comprises:
- identifying S150 one of the plurality of images 110 for assessing the vessel 120, based on a comparison of the calculated vessel overlap metrics.

In another example, a computer program product, is provided. The computer program product comprises instructions which when executed by one or more processors, cause the one or more processors to carry out a method identifying an angiographic image 110 for assessing a vessel 120. The method comprises:
- receiving S110 angiographic image data comprising one or more temporal sequences of images representing a flow of an injected contrast agent through the vessel 120; and
- for a plurality of images in the angiographic image data:
   segmenting S120 the vessel 120 in the image;
   analysing S130 the image to determine a contrast agent intensity INT variation along a portion of a length of the segmented vessel 140; and
   calculating S140, based on the contrast agent intensity INT variation, a vessel overlap metric for the vessel in the image, the vessel overlap metric quantifying a magnitude of overlap between the vessel 120 and one or more further vessels represented in the image; and

The method further comprises:
- identifying S150 one of the plurality of images 110 for assessing the vessel 120, based on a comparison of the calculated vessel overlap metrics.

The above examples are to be understood as illustrative of the present disclosure, and not restrictive. Further examples are also contemplated. For instance, the examples described in relation to the system 100, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for identifying an angiographic image (110) for assessing a vessel (120), the system comprising one or more processors (130) configured to:
receive (S110) angiographic image data comprising one or more temporal sequences of images representing a flow of an injected contrast agent through the vessel (120); and
for a plurality of images in the angiographic image data:
segment (S120) the vessel (120) in the image;
analyse (S130) the image to determine a contrast agent intensity (INT) variation along a portion of a length of the segmented vessel (140); and
calculate (S140), based on the contrast agent intensity (INT) variation, a vessel overlap metric for the vessel in the image, the vessel overlap metric quantifying a magnitude of overlap between the vessel (120) and one or more further vessels represented in the image; and
wherein the one or more processors (130) are further configured to:
identify (S150) one of the plurality of images (110) for assessing the vessel (120), based on a comparison of the calculated vessel overlap metrics.

2. The system according to claim 1, wherein the one or more processors (130) are configured to calculate the vessel overlap metric by: comparing the contrast agent intensity (INT) variation along the portion of the length of the segmented vessel (140) with one or more reference contrast agent intensity values.

3. The system according to claim 2, wherein the one or more processors (130) are further configured to:
identify one or more overlap regions (150) along the portion of the length of the segmented vessel (140) having contrast agent intensity values that exceed a reference contrast agent intensity value (THi); and
calculate the vessel overlap metric based on the identified one or more overlap regions (150).

4. The system according to claim 3, wherein the vessel overlap metric for the vessel in the image is calculated based on:
a total length (Li) of the one or more overlap regions for the vessel (120); or
a maximum contrast agent intensity value (INTₘₐₓ) within the one or more overlap regions for the vessel (120); or
a count of the number of overlap regions for the vessel (120).

5. The system according to claim 4, wherein the wherein the one or more processors (130) are configured to calculate the vessel overlap metric by further:
assigning confidence values to the contrast agent intensity values; and
wherein the vessel overlap metric for the vessel in the image is calculated based further on the confidence values.

6. The system according to any previous claim, wherein the one or more processors (130) are configured to identify (S150) the image for assessing the vessel (120) by selecting the image (110) having the lowest vessel overlap metric.

7. The system according to any previous claim, wherein the one or more processors (130) are further configured to:
display the identified image (110); and
indicate in the displayed image (110) a location of one or more overlap regions (150) of the vessel at which the vessel overlaps with one or more further vessels represented in the image.

8. The system according to any previous claim, wherein the one or more processors (130) are configured to segment the vessel in the images in the angiographic image data by:
determining a centerline (160) of the vessel in at least one of the images in the one or more temporal sequences of images; and
mapping the centerline (160) to one or more further images in the one or more temporal sequences of images.

9. The system according to claim 8, wherein the determining a centerline (160) comprises determining a centerline of the vessel in a plurality of images in the one or more temporal sequences of images, and wherein each centerline represents a different point in time in the cardiac cycle; and wherein the mapping the centerline (160) comprises mapping each centerline to the one or more further images in the one or more temporal sequences of images representing the same point in time in the cardiac cycle.

10. The system according to claim 8 or claim 9, wherein the one or more processors (130) are further configured to:
display an image in which the centerline (160) is determined;
receive input defining a start position (170), and an end position (170), of the portion of the vessel in the displayed image; and
map the start position (170) and the end position (180) from the displayed image to the one or more further images in the one or more temporal sequences of images to define the portion of the length of the segmented vessel (140) in said images.

11. The system according to any previous claim, wherein the one or more processors (130) are configured to analyse (S130) the images to determine a contrast agent intensity (INT) variation along a portion of a length of the segmented vessel (140), by:
determining a centerline (160) of the vessel in the image; and
determining image intensity values at a plurality of positions along the centerline (160).

12. The system according to claim 11, wherein the determining image intensity values at a plurality of positions along the centerline (160), comprises calculating an image intensity value at the plurality of positions along the centerline based on:
the image intensity value at the position; or
an average of the image intensity values within the segmented vessel (140) along a line transversely intersecting the centerline at the position; or
a maximum of the image intensity values within the segmented vessel (140) along a line transversely intersecting the centerline at the position.

13. The system according to any previous claim, wherein the one or more processors (130) are further configured to identify, from the angiographic image data, a plurality of images corresponding to a common point in time in the cardiac cycle and/or a plurality of images having contrast agent along at least the portion of the length of the segmented vessel (140), and wherein the operations of:
segmenting (S120) the vessel;
analysing (S130) the image to determine a contrast agent intensity (INT) variation along a portion of a length of the segmented vessel (140); and
calculating (S140), based on the contrast agent intensity (INT) variation, a vessel overlap metric for the vessel in the image, the vessel overlap metric quantifying a magnitude of overlap between the vessel (120) and one or more further vessels represented in the image;
are performed for the identified plurality of images.

14. The system according to any previous claim, wherein the one or more temporal sequences of images represent a flow of an injected contrast agent through a coronary vessel.

15. The system according to any previous claim, wherein the one or more processors (130) are further configured to:
calculate a value of a blood flow parameter for the vessel (120) using the image and/or calculate a value of a geometric measurement of the vessel (120) in the image.
